# DEMANDE DE BREVET EUROPEEN

(11) **EP 0 576 377 A1**
(43) Date de publication de la demande: **29.12.1993**
(21) Numéro de dépôt: 93420270.6
(22) Date de dépôt: 23.06.1993
(51) Int. Cl.: B01J 13/12

(54) **Microcapsules contenant au moins un principe actif, leurs applications et l'un de leurs procédés de préparation**

(30) Priorité: 24.06.1992 FR 9207992
(71) Demandeur: FLAMEL TECHNOLOGIES, Société Anonyme, F-69693 Venissieux Cédex (FR)
(72) Inventeur: Jorda, Rafael, 69110 STE FOY LES LYON (FR); Autant, Pierre, 03600 Commentry (FR); Rossin, René, 69600 Oullins (FR)
(74) Mandataire: Ropital-Bonvarlet, Claude

(57) **Abrégé**

- L'invention concerne des microcapsules contenant au moins un principe actif, caractérisées en ce qu'elles sont constituées par :
   . un noyau interne liquide, hydrophile et essentiellement non aqueux, formé d'une solution d'au moins un principe actif amphiphile et hydrosoluble dans au moins un solvant hydrophile non aqueux,
   . et par une paroi, enrobant ledit noyau interne et à base d'au moins un polymère ou copolymère,
   et également l'un de leurs procédés de préparation desdites microcapsules.
- Application à des systèmes de libération instantanée de principes actifs : e. g.articles d'hygiène, matériels et gants chirurgicaux, ...

## Description

La présente invention concerne des microcapsules contenant au moins un principe actif amphiphile et hydrosoluble, ainsi que leurs applications, en particulier dans le cadre d'un système de libération instantanée du principe actif, notamment par perforation, incision ou écrasement des microcapsules.

La présente invention est également relative à l'un des procédés envisageables pour la préparation desdites micro-capsules.

Les microcapsules sont des particules de forme sensiblement sphérique, dont la taille s'échelonne entre 1 et 2 000 micromètres, comprenant un coeur ou noyau interne constitué par une solution ou une dispersion de principe actif et par une enveloppe externe, généralement à base de polymères. De telles microcapsules sont de type réservoir.

Il existe, par ailleurs, des microcapsules de type matriciel, plus communément dénommées microsphères, dans lesquelles le principe actif est dispersé dans l'ensemble de la particule, au sein d'une matrice constituée par un polymère jouant le rôle d'excipient.

Le domaine de l'invention est celui des microcapsules de type réservoir.

Une grande variété de principes actifs peuvent être inclus dans les microcapsules. Il peut s'agir de médicaments, tels que les antibiotiques, les virucides ou les hormones, d'agents immunologiques, tels que les albumines, l'interféron, des antigènes ou des allergènes ou bien, encore, d'agents non biologiques, tels que des pesticides, des détergents, des pigments, des catalyseurs ou autres.

Ces microcapsules peuvent donc être utilisées dans de nombreux domaines, tels que la pharmacie, la bio-industrie, la cosmétologie, l'agro-alimentaire, l'agro-chimie, l'industrie papetière, etc ...

Dans les applications thérapeutiques, il est possible d'envisager deux modes de mise en oeuvre des microcapsules.

Le premier mode de mise en oeuvre est celui de la libération prolongée et contrôlée du principe actif dans le milieu extérieur aux microcapsules. Outre les contraintes de biocompatibilité et d'atoxicité, ce mode de mise en oeuvre suppose que l'enveloppe extérieure polymérique soit perméable au principe actif et qu'elle permette l'élution de celui-ci vers l'extérieur, selon une cinétique donnée et constante, en général d'ordre zéro. Il convient, également, que cette enveloppe extérieure conserve son intégrité structurelle face aux agressions chimiques, biochimiques ou mécaniques, qu'elle est susceptible de subir dans son environnement d'utilisation.

Dans le deuxième mode de mise en oeuvre des microcapsules, on exploite leur capacité à libérer massivement et instantanément le principe actif, suite à une contrainte mécanique, du type incision, perforation, écrasement, qui engendre la rupture, l'éclatement ou l'ouverture de leur enveloppe extérieure.

Pour ce genre d'application, qui intéresse plus particulièrement la présente invention, il importe que les microcapsules aient un seuil de rupture précisément adapté aux facteurs devant déclencher la libération du principe actif. L'enveloppe polymérique doit donc être suffisamment résistante pour éviter les libérations intempestives et suffisamment fragiles pour se rompre dans les circonstances voulues.

L'exemple de l'inclusion de microcapsules dans des gants chirurgicaux, susceptibles d'être accidentellement perforés par une aiguille, illustre ce deuxième mode de mise en oeuvre des microcapsules.

On connaît divers procédés permettant la micro-encapsulation de principes actifs de tous genres.

On a ainsi décrit, par exemple dans le brevet **US 4 675 189** et dans la demande de brevet **EP 0 052 610**, des techniques de séparation de phases ou coacervation, qui consistent à mettre en oeuvre un agent de coacervation, tel que les huiles minérales ou les huiles végétales.

De telles techniques sont très délicates à mettre en oeuvre, en raison du fait qu'elles donnent souvent lieu à la formation d'agglutinats, qui perturbent l'obtention de microcapsules.

Suivant d'autres techniques, dites de polymérisation ou de polycondensation interfaciale, on met en présence deux phases liquides non miscibles, dont l'une d'elles au moins contient un réactif polyfonctionnel apte à générer une polymérisation, comme par exemple un isocyanate. Il se produit une polymérisation à l'interface des deux liquides dans un milieu réactionnel qui peut être une émulsion, et on aboutit alors à la formation de microcapsules dont la paroi est formée d'un polymère de type polyamide, polyuréthanne, polyurée ou polyester.

Le brevet **BE 796 746** décrit l'encapsulation de principes actifs lipophiles par polymérisation d'un isocyanate, dans une émulsion huile dans eau et permettant d'obtenir des microcapsules dont la paroi est formée de polyurée.

Le brevet **FR 2 548 046** concerne un procédé analogue, permettant l'encapsulation de substances hydrosolubles à partir d'une émulsion eau dans huile.

La demande de brevet **EP 0 407 257** est relative à l'encapsulation de substances amphiphiles et hydrosolubles, telles que, par exemple, les ammoniums, mettant en oeuvre cette technique de polymérisation interfaciale.

Pour ces derniers composés, la polymérisation interfaciale n'est pas des plus adaptée. En effet, elle nécessite le recours à un noyau interne, support des substances amphiphiles, qui est à base de composés hydrophobes et qui contient d'importantes quantités de tensio-actifs nécessaires à la stabilisation du milieu. Or, ces derniers, ne sont pas sans perturber l'action et l'efficacité du principe actif. En outre, ces tensio-actifs ne permettent pas réellement d'éviter les phénomènes d'agglutination constatés lors de la préparation.

Il faut également signaler que, dans ces techniques, le noyau interne ne doit pas contenir de produits susceptibles de contrarier la polymérisation, par exemple en réagissant avec les agents de polymérisation, tels que les isocyanates. On sait, notamment, que les polyols, du type polyéthylène glycol, propylène glycol ou glycérol, sont, à ce titre, inappropriés.

Enfin, les microcapsules obtenues par ce procédé présentent une paroi mécaniquement inadaptée à une utilisation dans un système de libération massive et instantanée de principe actif.

D'autres techniques d'obtention de microcapsules reposent sur la formation d'une simple émulsion huile dans eau ou d'une double émulsion eau dans huile dans eau et sur l'extraction du solvant du polymère, de manière à solidifier celui-ci. Le polymère forme en fait la phase dite "huileuse" de ces émulsions.

Dans ce type de techniques, le principe actif est, soit dispersé dans la phase lipophile contenant le polymère en solution dans un solvant organique (émulsion simple huile dans eau), soit contenu dans la phase aqueuse interne E₁ d'une double émulsion eau dans huile dans eau (E₁/H/E₂).

La demande de brevet international WO 90/13 361 décrit un procédé mettant en oeuvre une double émulsion, dans lequel une solution de polymère dans un solvant organique est mise en contact avec un principe actif non amphiphile, éventuellement en milieu aqueux, de manière à former une première émulsion eau dans huile, dite émulsion primaire (E₁/H). Cette dernière est ensuite introduite dans un milieu dispersant constitué, de préférence, essentiellement d'eau et dans lequel se forme une émulsion secondaire eau dans huile dans eau (E₁/H/E₂).

Selon ce procédé ou d'autres du type double émulsion (E₁/H/E₂), l'eau est préférentiellement employée pour constituer la phase aqueuse interne de l'émulsion primaire E₁. L'eau présente l'avantage d'être une matière première économique, biocompatible, atoxique et idéale pour la formation d'émulsions. De façon appréciable, elle est également, dans la plupart des cas, inerte et compatible vis-à-vis des principes actifs amphiphiles et hydrosolubles, plus particulièrement concernés par l'invention.

Mais son utilisation n'est pourtant pas sans poser de problèmes au regard de l'application des microcapsules à des systèmes de libération massive et instantanée du principe actif, dans lesquels le principe actif doit être très rapidement disponible et efficace vis-à-vis de la cible.

En effet, dans cette application, les microcapsules sont noyées à l'intérieur d'un élastomère qui forme la matière constitutive de l'article considéré, par exemple des gants chirurgicaux. Or, la réticulation de l'élastomère, qui intervient lors de l'élaboration de tels articles, s'opère à haute température, de l'ordre de 120°C. Il s'ensuit que le noyau interne aqueux des microcapsules contenant le principe actif s'évapore ce qui enlève beaucoup de son efficacité au principe actif après rupture de la paroi.

Dans cet état de fait, l'un des buts essentiels de la présente invention est de fournir des microcapsules renfermant au moins un principe actif et constituées par un noyau interne contenant ledit principe actif et enrobé dans une paroi à base de polymère, le principe actif encapsulé étant amphiphile et hydrosoluble.

Un autre but de l'invention est de fournir des microcapsules qui soient aptes à être appliquées dans des systèmes à libération instantanée et massive de principes actifs et, en particulier, dans les gants chirurgicaux.

Un autre but de l'invention est de fournir des microcapsules contenant un principe actif amphiphile, qui soient, notamment, de qualité constante, aisées à obtenir, faites de matériaux compatibles avec le principe actif et stables à la température.

Ces buts et d'autres sont atteints par les microcapsules conformes à l'invention qui concernent, à titre de produit industriel nouveau, des microcapsules contenant au moins un principe actif, caractérisées en ce qu'elles sont constituées par:
- un noyau interne liquide, hydrophile et essentiellement non aqueux, formé d'une solution d'au moins un principe actif amphiphile et hydrosoluble dans au moins un solvant hydrophile non aqueux,
- et par une paroi, enrobant ledit noyau interne et à base d'au moins un polymère ou copolymère.

La présence d'un noyau interne liquide hydrophile et non aqueux contenant au moins un principe actif amphiphile et hydrosoluble en solution dans au moins un solvant hydrophile non aqueux, constitue une disposition tout à fait originale de l'invention.

Au sens de la présente invention, le qualificatif "essentiellement" signifie que le solvant hydrophile non aqueux est prépondérant dans la phase solvant considérée et, de préférence, que la proportion d'eau dans la phase solvant est inférieure à 50 % en poids.

Ces microcapsules sont de dimensions homogènes et ont un diamètre moyen compris entre 50 et 2 000 µm, selon les conditions d'obtention. Elles se prêtent très bien à des applications du type système à libération instantanée de principe actif. Elles sont, en effet, suffisamment élastiques pour n'être écrasées et ne libérer le principe actif qu'elles contiennent qu'après l'exercice d'une pression importante, et non lors d'une simple manipulation. Elles sont parfaitement stables au stockage à l'air. On n'observe pas d'évaporation du solvant non aqueux du noyau interne. Par ailleurs, on montre que ces microcapsules sont stables à haute température (100° C). L'évaporation du solvant non aqueux hydrophile se produit de manière limitée.

De telles microcapsules sont particulièrement bien adaptées à un usage externe et peuvent être incorporées, de façon non limitative, à des papiers, des textiles (tissés ou non tissés), y compris des objets de pansement (pansements, compresses) et des champs opératoires, des éponges et des matériaux à base de polymères, en particulier d'élastomères (gants à usage médical ou chirurgical, préservatifs, etc ...), à des brosses à ongles (brosses chirurgicales, par exemple), à des poudres désinfectantes, etc ...

Le principe actif est un composé amphiphile et hydrosoluble formé, de préférence, par un ammonium quaternaire. Plus préférentiellement encore, le composé amphiphile est le diméthyldidécylammonium.

Au sens de la présente invention, on entend par diméthyldidécylammonium, aussi bien le produit de base que ses sels (halogénure, dihalogénure, sels organiques par exemple).

On sait, en effet, que les ammoniums quaternaires, du type du diméthyldidécylammonium, présentent un intérêt particulier en applications locales comme bactéricide, fongicide et virucide. A titre illustratif, on peut signaler que le diméthyldidécylammonium possède une activité efficace à l'encontre du virus **HIV**.

Contre toute attente, l'invention permet de s'affranchir de l'utilisation de l'eau pour le noyau interne des microcapsules qui se prêtent bien, de par leur dureté, à des applications à titre de systèmes à libération instantanée et massive de principes actifs par perforation ou écrasement.

Les produits mis en oeuvre dans la préparation des microcapsules selon l'invention n'altèrent pas l'activité du principe actif. Qui plus est, celui-ci est un composé amphiphile hydrosoluble, pourtant connu pour favoriser les émulsions huile dans eau, alors que la micro-encapsulation requiert des émulsions du type eau dans huile dans eau.

Suivant une autre modalité avantageuse de l'invention, la concentration en principe actif dans les microcapsules est comprise entre 0,01 et 50 % (poids/poids), de préférence entre 0,1 et 15 % (poids/poids) et, plus préférentiellement encore entre 0,5 à 5 % (poids/poids).

Pour améliorer la stabilité thermique du noyau interne des microcapsules, il est avantageux que le solvant non aqueux hydrophile possède, à pression atmosphérique normale, une température d'ébullition supérieure à 100° C, de préférence à 120° C et, plus particulièrement encore, à 150° C. Il est présent dans les microcapsules à raison de 10 à 60 % (poids/poids) environ.

Conformément à une disposition préférée de l'invention, le solvant non aqueux hydrophile est un composé ou un mélange de composés choisis dans la famille des polyols et/ou des polyéthers hydroxylés, de préférence parmi la liste non limitative de composés suivants :
- 1-2 propanediol,
- polyéthylèneglycol de masse moléculaire, de préférence, comprise entre 100 et 700,
- glycérol, ce dernier produit étant particulièrement préféré.

Le polymère est présent dans les microcapsules à raison de 20 à 90 % (poids/poids), de préférence de 40 à 70 %.

Conformément à une autre modalité intéressante de l'invention, ce polymère est choisi parmi les produits suivants, seuls ou en mélanges :
copolymères d'éthylène et d'acétate de vinyle (EVA), polystyrènes, polyesters,
silicones, polymères et copolymères à base de chlorures de vinylidène
et/ou de chlorures de vinyle, polycarbonates.

En pratique, on préfère (sans que cela ne soit limitatif), employer un copolymère d'éthylène et d'acétate de vinyle (EVA), dont le pourcentage en acétate de vinyle est, de préférence, compris entre 25 et 50 % et, plus préférentiellement encore, entre 28 et 40 %.

La présente invention propose également un nouveau procédé de préparation des susdites microcapsules, du type de ceux consistant à :
- réaliser une émulsion primaire, du type émulsion eau dans huile (E₁/H), à l'aide d'une première phase E₁ hydrophile et d'une seconde phase H lipophile constituée par une solution du polymère dans au moins un solvant organique,
- mettre cette émulsion primaire en présence d'une troisième phase E₂ hydrophile, de façon à former une émulsion secondaire du type émulsion eau dans huile dans eau (E₁/H/E₂),
- et éliminer le solvant du polymère, de manière à solidifier celui-ci.
   Ce nouveau procédé est caractérisé en ce que :
   - on prépare l'émulsion primaire (E₁/H) au moyen d'une solution ou d'une dispersion A du principe actif dans un solvant non aqueux et hydrophile (première phase hydrophile E₁) et d'une composition B constituée par une solution d'au moins un polymère ou copolymère dans au moins un solvant organique (seconde phase H lipophile),
   - on met cette émulsion primaire en présence d'une fraction C₁ d'une composition hydrophile liquide C (troisième phase E₂ hydrophile), dite de dispersion et d'extraction, et contenant au moins deux solvants Sc₁, Sc₂ miscibles entre eux, l'un des deux solvants, Sc₁, étant miscible avec le solvant du polymère, l'autre Sc₂, étant non miscible avec ledit solvant et le polymère étant insoluble dans Sc1 et/ou Sc₂, de manière à former une émulsion secondaire E₁/H/E₂ et, sensiblement simultanément, à extraire au moins une partie du solvant du polymère,
   - et on ajoute une fraction C₂ de la composition C pour compléter l'extraction et pour entraîner la prise en masse dudit polymère.

Par insoluble, on entend, au sens de la présente invention, une solubilité, de préférence inférieure à 1 g/l à une température de 25° C.

Conformément à l'invention, deux liquides sont considérés comme miscibles si, et seulement si, ils forment, par mélange, une phase unique dans les proportions de l'utilisation.

Il est préférable, d'une part, que le principe actif soit insoluble dans le solvant du polymère, de manière à éviter sa migration dans la phase lipophile et, d'autre, part, que le solvant non aqueux hydrophile ne soit pas miscible avec le solvant du polymère, de telle sorte que l'émulsion primaire E₁/H puisse se former.

La composition C dispersante et extractive contient au moins deux solvants Sc₁, Sc₂, dans lesquels le polymère est insoluble.

Sc₁ est apte à entraîner le solvant du polymère, alors que Sc₂ est non miscible avec ledit solvant du polymère.

Le fait de prévoir deux solvants, dont un seulement est actif vis-à-vis du solvant à extraire, permet de maîtriser la vitesse d'extraction et de surmonter les problèmes d'agglutination très fréquents dans cette technique délicate de la double émulsion.

Sc₁ et Sc₂ sont présents dans la composition C selon un ratio Sc₁/Sc₂ choisi de telle sorte que la fraction du polymère, extraite par la composition C, soit comprise entre 75 et 100 %, de préférence supérieure à 95 % en poids par rapport à la quantité initiale totale en solvant du polymère.

De préférence, le ratio Sc₁/Sc₂ est compris entre 10 et 40 % et, plus préférentiellement encore, entre 15 et 30 % (volume/volume).

Les solvants Sc₁ et Sc₂ sont des solvants organiques polaires.

Sc₁ peut être choisi dans les familles des alcools, polyols et polyéthers hydroxylés.

Sc₂ peut, quant à lui, appartenir aux familles de composés chimiques suivantes : polyols, polyéthers hydroxylés.

Pour prévenir mieux encore les problèmes d'agglutination, il est préférable d'ajouter à la composition C un agent dispersant et/ou un agent lubrifiant, tel que le talc.

Selon une disposition avantageuse du procédé suivant l'invention, l'ajout de la composition C à l'émulsion primaire E₁/H est effectué de manière fractionnée.

En pratique, et sans que cela ne soit limitatif, la composition C est divisée en trois sous-compositions C₁, C₁' et C₂, incorporées successivement à l'émulsion primaire E₁/H.

A titre d'exemple, la composition C₁ représente environ 70 % en volume de la composition C, la sous-composition C₁' environ 10 % en volume, cette sous-composition C₁' comprenant, avantageusement, un agent lubrifiant, tel que le talc, et la composition C₂ environ 20 % en volume de la compositon C.

L'agent lubrifiant, éventuellement introduit dans la composition C, est présent à une concentration de 1 à 7 % (poids/poids) environ par rapport aux microcapsules.

Conformément à une variante de ce procédé, l'élimination du solvant du polymère associe la technique d'extraction de solvant décrite ci-dessus et celle d'évaporation.

L'élimination de solvant du polymère par évaporation dans les procédés de micro-encapsulation du type double émulsion E₁/H/E₂ est une technique connue en elle-même. Il est envisageable de la mettre en oeuvre seule sans extraction de solvant.

L'invention sera mieux comprise et ses avantages, ainsi que ses variantes de mise en oeuvre, ressortiront bien des exemples qui suivent.

### EXEMPLES

Les exemples 1 à 9 illustrent le premier procédé de préparation de microcapsules conformes à l'invention.
Les exemples 10 et 11 illustrent le second procédé.
Les exemples 12 à 15 illustrent le troisième procédé.

### EXEMPLE 1 :

**A - MATÉRIAUX** **:**
   **1. Le principe actif :**
      Le principe actif encapsulé est le chlorure de diméthyldidécylam monium ("BARDAC 22" LONZA - FRANCE). C'est un sel d'ammonium quaternaire, qui présente donc toute les propriétés de cette classe chimique :
      - C'est un tensioactif cationique hydrosoluble, dont le pouvoir mouillant est important. Il est incompatible, notamment, avec les tensioactifs anioniques, tels que les savons, les cations divalents, les nucléinates d'argent et les proténates d'argent. Il s'adsorbe sur les élastomères.
      - C'est un antiseptique et un désinfectant :
         . très actif sur les bactéries à Gram**⁺,**
         . actif sur les bactéries à Gram⁻, les champignons et les virus (tétrovirus, en particulier l'HIV),
         . inactif sur les mycobactéries et les spores.

      Il peut s'appliquer sur la peau (abord chirurgical, mains, plaies, brûlures) et sur la muqueuse conjonctivale. La solution aqueuse commerciale est à 60 %. On mesure la viscosité de la solution de "**BARDAC 22**" à l'aide d'un viscosimètre à capillaire à 20° C (tube Ubbelohde). On obtient, pour des solutions à 20 %, 30 % et 40 % des viscosités ayant pour valeurs respectives 28, 36, et 47 centipoises à 20° C.
   **2. Le polymère :**
      La paroi des microcapsules est constituée d'EVA (éthylène/vinyleacétate copolymère) contenant 40 résidus vinyleacétate pour 100 résidus totaux ("ELVAX 40 W").
   **3. Les compositions A, B et C :**
      - Composition A :
         . glycérol (99 % JANSSEN CHIMICA) 13,25 g
         . BARDAC 22 (LONZA) à 60 % dans l'eau de diméthyldidécylammonium 2,65 g
         . Erythrosine B (JANSSEN CHIMICA) 0,10 g
      - Composition B :
         . cyclohexane (LAURYLAB) 69 ml
         . ELVAX 40 W (DUPONT DE NEMOURS) 12 g
      - Composition C :
         . 1-2 propane diol (99 % JANSSEN CHIMICA) 454 cc
         . Butanol 1 (LAURYLAB) 131 cc

      La composition C donne naissance à trois sous-compositions :
      .. C₁ ou pied de cuve : C 350 cc
      .. C₁' ou lubrifiante : C 50 cc
         talc officinal 4 g
      .. C₂ ou ajout 185 cc
**B - PROTOCOLE** :
   **1. Emulsion primaire :**
      Dans un erlermeyer de 150 ml contenant la composition B, ajouter sous agitation la solution A. Laisser agiter pendant 30 secondes à une vitesse de 800 tr/min.
      L'émulsion obtenue a une taille de 5 à 100 µm.
   **2. Emulsion secondaire :**
      Dans un réacteur à fond rond de un litre, équipé d'une agitation et d'une ampoule de coulée, verser rapidement l'émulsion primaire dans la sous-composition C₁ ou pied de cuve . La vitesse d'agitation est de 500 tr/min. Puis verser rapidement la sous-composition C₁' (ou lubrifiante).
      On commence l'ajout de la solution C₂. L'ajout dure de 20 à 30 min.
      On laisse l'agitation pendant 15 min après la fin de coulée de la sous-composition C₂ pour compléter l'extraction du cyclohexane.
   **3. Traitement des microcapsules :**
      - filtration sur une toile de polyéthylène de 90 µm,
      - lavage par deux fois avec 150 ml d'éthanol absolu,
      - séchage sous courant d'air.
**C - RÉSULTATS :**
   On obtient des microcapsules contenant 53 % de liquide hydrophile, 1,8 % de diméthyldidécylammonium par rapport au poids de microcapsules.
   Les microcapsules ne sèchent pas à l'air et ne se vident pas. Pas d'exsudation du liquide interne.
   Après 18 h à 40° C, il reste 37 % de liquide.
   Après 56 h à 40° C, il en reste encore 35 %.
   Après 2 h à 100° C, il reste 23 % de liquide à l'intérieur des microcapsules.
   Taille moyenne des capsules : diamètre N 400 µm.

### EXEMPLE 2 :

On prépare une composition A contenant 6,6 g de glycérol et 1,3 g de BARDAC 22.
On prépare une compositon B contenant 6 g d'ELVAX 240 (DUPONT DE NEMOURS) et 53,6 g de cyclohexane.
En appliquant la méthode décrite dans l'exemple 1, les microcapsules fabriquées présentent une taille légèrement inférieure, diamètre de l'ordre de N 300 µm, à celles obtenues avec l'exemple 1.
Les microcapsules contiennent 39 % de liquide hydrophile.

### EXEMPLE 3 :

Le glycérol de la composition A est remplacé par 13,25 g de polyéthylène glycol 400 (JANSSEN).
Par la méthode décrite dans l'exemple 1, on obtient des microcapsules similaires à celles fabriquées précédemment.

### EXEMPLE 4 :

Dans la composition C, on remplace le butanol-1 par 120 cc de propanol-2 (PROLABO).
La compositon C₂ (ajout) a un volume de 174 cc.
La méthode est identique à l'exemple 1 et les microcapsules obtenues ont une taille légèrement supérieure à celles fabriquées dans l'exemple 1.

### EXEMPLE 5 :

La composition B contient 12 g d'ELVAX 46 L (DUPONT DE NEMOURS) et 53,6 g de cyclohexane.
D'après la méthode décrite dans l'exemple 1, les microcapsules obtenues ont une forme plus allongée mais leur contenu est identique à celles fabriquées dans l'exemple 1. La stabilité des microcapsules à 100° C est meilleure que celle mesurée avec le copolymère ELVAX 40 W.

### EXEMPLE 6 :

On procède comme dans l'exemple 1. Toutefois, les 12 g de ELVAX 40 W sont remplacés par 6 g d'ELVAX 170 et on utilise 53,6 g de cyclohexane. On utilise, également, la moitié de la quantité de solution A.
Les microcapsules obtenues ont une forme peu sphérique et une taille allant de 200 µm à 2 000 µm.
Leur contenu est similaire à celui décrit dans l'exemple 1.
L'ELVAX 170 a une meilleure tenue en température que l'ELVAX 40 W. On observe moins de fluage du polymère à 100° C.

### EXEMPLE 7 :

Les mêmes matériaux et le même protocole que ceux de l'exemple 1 sont mis en oeuvre, à la différence près que, dans la composition B, le cyclohexane est remplacé par un mélange 50/50 de cyclohexane et d'hexane.
L'émulsion primaire obtenue est centrée autour de 10 µm.
Les microcapsules obtenues sont semblables à celles de l'exemple 1.

### EXEMPLE 8 :

Dans cet exemple, le procédé change légèrement.
La composition C₁ n'est maintenant constituée que par 400 cc de 1-2 propanediol.
Elle ne contient plus de butanol-1.
La composition C₁' :
- 1-2 propanediol 54 cc
- talc officinal 4 g

La compositon C₂ est constituée de la totalité du butanol. 1 mis en oeuvre, soit 131 cc.
La seconde émulsion est faite dans la composition C₁ et l'extraction du cyclohexane ne commence qu'avec la coulée de la composition C₂. Cette coulée se fait en 1 h et l'ajout de la composition C₁' s'opère au bout d'une demi-heure de coulée.
Les microcapsules sont plus grosses que dans l'exemple 1 (de 600 µm à 2 000 µm) et contiennent autant de liquides hydrophiles (≃ 50 %) et de diméthyldidécylammonium (≃ 1 %).

### EXEMPLE 9 :

Le même protocole et les mêmes matériaux que ceux de l'exemple 1 sont mis en oeuvre, à la différence près que le glycérol de la compositon A est remplacé par du 1-2 propanediol.
Les microcapsules obtenues font de 500 µm à 800 µm, contiennent environ 50 % de liquides internes.
Après 1 h à 40° C, 47 % de 1-2 propanediol sont trouvés en phase interne.
Après 16 h à 40° C, on trouve encore 39 % de liquide interne.
Ce pourcentage diminue très peu par la suite.

### EXEMPLE 10 :

Des microcapsules, de type réservoir, sont réalisées par un procédé par évaporation de solvant. Dans ce procédé, la phase dispersante n'a pas de capacité d'extraction vis-à-vis du solvant du polymère enrobant.
Une émulsion primaire est préparée par dispersion d'une phase interne hydrophile dans une phase externe contenant le polymère en solution organique. Cette émulsion primaire est ensuite dispersée dans une phase dispersante. Le solvant organique est éliminé par évaporation sous vide.
Emulsion primaire:
- phase interne (E₁):
   . glycérol 18,3 g
   . BARDAC 2270 E
      (70 % de diméthyldidécylammonium) 9,2 g
   . solution acqueuse à 10 % poids de RHODOVIOL 25/140 (alcool polyvinylique RHONE POULENC 9,2 g
- phase externe (H):
   . ELVAX 40 W 18,3 g
   . dichlorométhane 165,1 g

Cette émulsion primaire est dispersée dans :
- phase dispersante (E₂) :
   . eau déminéralisé 375 g
   . glycérol 375 g
   . BARDAC 2270 E 30 g
   . antimousse silicone RHODORSIL 426 R (RHONE POULENC) 2 ml

En fin de dispersion on ajoute :
. talc 15 g

Le dichlorométhane est évaporé sous pression réduite à température ambiante, selon le profil de pression suivant :
. sous 460 mm Hg 3 min
. sous 300 mm Hg 5 min
. sous 160 mm Hg 8 min
. sous 30 mm Hg 1 h 20 min

On recueille environ 30 g de microcapsules de diamètre compris entre 100 et 315 µm, contenant 6 % de chlorure de diméthyldidécylammonium et 26 % de phase liquide encapsulée dont 62 % de glycérol.

### EXEMPLE 11 :

Dans cet exemple, on utilise un procédé par réticulation d'une résine silicone. Un solvant organique est utilisé pour diminuer la viscosité de la résine lors de l'introduction dans le réacteur. Le solvant est immédiatement éliminé par évaporation sous pression réduite et la réticulation de la résine est ensuite réalisée à une température comprise entre 50 et 95° C.
Emulsion primaire :
- phase interne (E₁) :
   . glycérol 5 g
   . BARDAX 2270 E 2,5 g
   . solution aqueuse à 10 % poids de RHODOVIOL 25/140 2,5 g
- phase externe (H) :
   . résine silicone RTV 70141 A (RHONE POULENC) 8 g
   . résine silicone RTV 70141 B (RHONE POULENC) 2 g
   . dichlorométhane 15 g
   . dipélargonate de propylène glycol (GATTEFOSSE) 0,4 g

Cette émulsion primaire est dispersée dans :
- phase dispersante (E₂) :
   . eau déminéralisé 360 g
   . solution aqueuse à 10 % poids de RHODOVIOL 25/140 40 g

En fin de dispersion on ajoute :
. silice (TixOSil 331) en suspension dans 5 g
. eau déminéralisée 60 g

Le solvant est éliminé par distillation sous vide (sous 20 à 40 mm Hg), puis la réaction de réticulation est réalisée en 1 h par chauffage au-dessus de 50° C.

### EXEMPLE 12 :

Dans cet exemple, on réalise des microcapsules de type réservoir, par une méthode combinant, simultanément, l'extraction et l'évaporation du solvant de la phase polymère enrobante.
Une émulsion primaire composée d'une phase interne hydrophile et d'une phase externe contenant un polymère en solution organique est dispersée dans une phase dispersante présentant une capacité d'extraction vis-à-vis du solvant du polymère enrobant. Cette capacité d'extraction est initialement limitée par saturation partielle en solvant. L'élimination progressive du solvant est ensuite poursuivie par évaporation sous pression réduite.
Emulsion primaire :
- phase interne (E₁) :
   . glycérol 10 g
   . solution aqueuse à 10 % poids de RHODOVIOL 25/140 5 g
- phase externe (H) :
   . ELVAX 40 W 10 g
   . dichlorométhane 90 g

Cette émulsion primaire est dispersée dans :
- phase dispersante :
   . eau déminéralisée 173 g
   . polyéthylène glycol PEG 200 403,5 g
   . dichlorométhane 173 g

En fin de dispersion on ajoute :
. talc (LUZENAC, STEAMIC OOS) 7,5 g

Le dichlorométhane est évaporé sous vide à température ambiante, en 1 h 30 selon le profil de pression suivant :
. sous 610 mm Hg 15 min
. sous 460 mm Hg 15 min
. sous 360 mm Hg 15 min
. sous 260 mm Hg 30 min
. sous 160 mm Hg 15 min

Le produit est récupéré par filtration, lavé à l'eau déminéralisée et séché par fluidisation à température ambiante.
On obtient ainsi 16 g de microcapsules de type réservoir, de diamètre environ 400 microns, dont la composition est la suivante :
. teneur en chlorure de diméthyldidécylammonium 1 %
. teneur en phase externe (enrobant) 57,8 %
   dont 96,5 % d'ELVAX et 3,5 % de talc
. teneur en phase interne 42,2 %
   dont 18 % d'eau et 82 % d'un mélange glycérol et PEG

### EXEMPLE 13 :

Dans cet exemple on procède selon la méthode décrite à l'exemple 12, mais en limitant la capacité d'extraction du PEG par du glycérol et en incorporant une petite quantité de BARDAC en phase dispersante, afin de limiter les échanges avec la phase interne hydrophile.
Emulsion primaire:
- phase interne (E₁) :
   . glycérol 11,25 g
   . BARDAC 2270 E 5,6 g
   . solution aqueuse à 10 % poids de RHODOVIOL 25/140 5,6 g
- phase externe (H) :
   . ELVAX 40 W 11,25 g
   . dichlorométhane 101,25 g

Cette émulsion primaire est dispersée dans :
- phase dispersante (E₂) :
   . eau déminéralisée 510 g
   . glycérol 127,5 g
   . PEG 300 112,5 g
   . BARDAC 2270 7,5 g

En fin de dispersion on ajoute :
. talc 7,5 g

Le dichlorométhane est évaporé sous pression réduite à température ambiante, selon le profil de pression décrit à l'exemple 12.
On recueille, après filtration, lavage et séchage, environ 14 g de microcapsules de type réservoir, de diamètre compris entre 100 et 400 microns, contenant, selon les conditions opératoires, entre 3 et 4,5 % de chlorure de diméthyldidécylammonium et présentant entre 30 et 33 % de phase liquide encapsulée constituée par 50 à 64 % de glycérol.

### EXEMPLE 14 :

Selon la méthode de l'exemple 13, on réalise des microcapsules de type réservoir en remplaçant l'ELVAX 40 W par du polystyrène (GEDEX 6519 JA 200 NORSOLOR).
Emulsion primaire :
- phase interne (E₁) :
   . glycérol 5 g
   . BARDAC 2270 E 2,5 g
   . solution aqueuse à 10 % poids de RHODOVIOL 25/140 2,5 g
- phase externe (H) :
   . GEDEX 6519 JA 200 10 g
   . dichlorométhane 90 g

Cette émulsion primaire est dispersée dans :
- phase dispersante :
   . eau déminéralisée 460 g
   . glycérol 115 g
   . PEG 300 100 g
   . BARDAC 2270 E 7 g

En fin de dispersion on ajoute :
. talc 7 g

Le dichlorométhane est évaporé sous pression réduite à température ambiante, selon le profil de pression décrit à l'exemple 12.
On recueille ainsi des microcapsules de 100 à 200 microns de diamètre, contenant 18 % de phase liquide encapsulée, dont 69 % de glycérol.

### EXEMPLE 15 :

Selon la méthode décrite par l'exemple 13, on réalise des microcapsules de type réservoir en remplaçant l'ELVAX 30 W par un mélange de polyesters (copolymères DYNAPOL L 206/1 et DYNAPOL LS 415 de HULS).
Emulsion primaire :
- phase interne (E₁) :
   . glycérol 10 g
   . BARDAC 2270 E 5 g
   . solution aqueuse à 10 % poids de RHODOVIOL 25/140 5 g
- phase externe (H) :
   . DYNAPOL L 206/1 14 g
   . DYNAPOL LS 415 6 g
   . dichlorométhane 80 g

Cette émulsion primaire est dispersée dans :
- phase dispersante (E₂) :
   . eau déminéralisée 460 g
   . glycérol 115 g
   . PEG 300 100 g
   . BARDAC 2270 E 7 g

En fin de dispersion on ajoute :
. talc 7 g

Le dichlorométhane est évaporé sous pression réduite à température ambiante, selon le profil de pression décrit à l'exemple 12.
On recueille ainsi des microcapsules contenant 26 % de phase liquide encapsulée dont 52 % de glycérol.

## Revendications

**1 -** Microcapsules contenant au moins un principe actif, caractérisées en ce qu'elles sont constituées par :
- un noyau interne liquide, hydrophile et essentiellement non aqueux, formé d'une solution d'au moins un principe actif amphiphile et hydrosoluble dans au moins un solvant hydrophile non aqueux,
- et par une paroi, enrobant ledit noyau interne et à base d'au moins un polymère ou copolymère.

**2 -** Microcapsules selon la revendication 1, caractérisées en ce que le solvant hydrophile non aqueux possède une température d'ébullition, à pression atmosphérique normale, supérieure à 100°C, de préférence à 120° C et, plus préférentiellement encore, supérieure à 150° C.

**3 -** Microcapsules selon la revendication 1 ou 2, caractérisées en ce que le solvant hydrophile non aqueux est un composé ou un mélange de composés choisis dans la famille des polyols et/ou des polyéthers hydroxylés, de préférence parmi la liste non limitative de produits suivants :
- 1-2 propanediol,
- polyéthylène glycol de masse moléculaire de préférence comprise entre 100 et 700,
- glycérol, ce dernier produit étant particulièrement préféré.

**4 -** Microcapsules selon l'une quelconque des revendications 1 à 3, caractérisées en ce que le principe actif amphiphile est un ammonium quaternaire, de préférence, le diméthyldidécylammonium.

**5 -** Microcapsules selon l'une quelconque des revendications 1 à 4, caractérisées en ce que le principe actif est présent à une concentration comprise entre 0,01 et 50 % (poids/poids), de préférence entre 0, 1 et 15 % (poids/poids) et, plus préférentiellement encore, de 0,5 à 5 % (poids/poids).

**6 -** Microcapsules selon l'une quelconque des revendications 1 à 5, caractérisées en ce que le polymère est choisi parmi les produits suivants, seuls ou en mélanges :
copolymères d'éthylène et d'acétate de vinyle (EVA), polystyrènes, polyesters,
silicones, polymères ou copolymères à base de chlorures de vinylidène et/ou de vinyle, polycarbonates ;
les copolymères d'éthylène et d'acétate de vinyle (EVA) étant préférés.

**7 -** Microcapsules selon l'une quelconque des revendications 1 à 6, caractérisées en ce que le polymère est présent à raison de 20 à 90 % (poids/poids), de préférence de 40 à 70 % (poids/poids) et, plus préférentiellement encore, de 40 % (poids/poids) environ.

**8 -** Procédé de préparation de microcapsules contenant au moins un principe actif et constituées par :
- un noyau interne liquide, hydrophile et essentiellement non aqueux, formé d'une solution d'au moins un principe actif amphiphile et hydrosoluble dans au moins un solvant hydrophile non aqueux,
- et par une paroi, enrobant ledit noyau interne et à base d'au moins un polymère ou copolymère,
dans lequel :
- on réalise une émulsion primaire du type émulsion eau dans huile (E₁/H) à l'aide d'une première phase (E₁) hydrophile et d'une seconde phase (H) lipophile constituée par une solution du polymère dans au moins un solvant organique,
- on met cette émulsion primaire en présence d'une troisième phase (E₂) hydrophile de façon à former une émulsion secondaire du type émulsion eau dans huile dans eau (E₁/H/E₂),
- et on élimine le solvant du polymère de manière à solidifier celui-ci,
caractérisé en ce qu'il consiste à :
- effectuer l'émulsion primaire au moyen d'une solution ou d'une dispersion (A) de principe actif dans un solvant non aqueux et hydrophile et d'une composition (B) constituée par une solution d'au moins un polymère ou copolymère dans au moins un solvant organique,
- mettre cette émulsion primaire en présence d'une fraction (C₁) d'une composition hydrophile liquide (C) de dispersion et d'extraction contenant au moins deux solvants (Sc₁, Sc₂) miscibles entre eux, le polymère étant insoluble dans (Sc₁ et/ou Sc₂), l'un des deux solvants (Sc₁) étant miscible avec le solvant du polymère et l'autre (Sc₂) étant non miscible avec ledit solvant, de manière à former l'émulsion secondaire et, sensiblement simultanément, à extraire au moins une partie du solvant du polymère,
- et ajouter une fraction (C₂) de la composition (C) pour compléter l'extraction et pour entraîner la prise en masse dudit polymère et, par suite, la formation des microcapsules.

**9** **-** Procédé selon la revendication 8, caractérisé en ce que les deux solvants (Sc₁, Sc₂) de la composition (C) sont présents dans un ratio (Sc₁/Sc₂) choisi de telle sorte que la fraction du solvant du polymère extraite par la composition C soit comprise entre 75 et 100 %, de préférence supérieure à 95 %, en poids par rapport à la quantité totale initiale en solvant du polymère.

**10 -** Procédé selon la revendication 8 ou la revendication 9, caractérisé en ce que l'ajout de la composition (C) à l'émulsion primaire (E₁/H) est effectué de manière fractionnée.

**11 -** Procédé selon l'une quelconque des revendications 8 à 10, caractérisé en ce que la composition (C) comprend un agent dispersant et/ou un agent lubrifiant, tel que le talc.

**12 -** Procédé de préparation selon l'une quelconque des revendications 8 à 11, caractérisé en ce que l'élimination du solvant du polymère associe l'extraction de solvant et l'évaporation.

**13 -** Papiers ou textiles, caractérisés en ce qu'il contiennent des microcapsules selon l'une quelconque des revendications 1 à 7.

**14 -** Objets de pansements, caractérisés en ce qu'ils contiennent des microcapsules selon l'une quelconque des revendications 1 à 7.

**15 -** Produits à base de polymères, en particulier délastomères, caractérisés en ce qu'il contiennent des microcapsules selon l'une des revendications 1 à 7.

**16 -** Gants à usage médical ou chirurgical, caractérisés en ce qu'ils contiennent des microcapsules selon l'une quelconque des revendications 1 à 7.

**17 -** Préservatifs, caractérisés en ce qu'ils contiennent des microcapsules selon l'une quelconque des revendications 1 à 7.

**18 -** Poudres désinfectantes, caractérisées en ce qu'elles contiennent des microcapsules selon l'une quelconque des revendications 1 à 7.

**19 -** Eponges, caractérisées en ce qu'elles contiennent des microcapsules selon l'une quelconque des revendications 1 à 7.

**20 -** Brosses à ongles, caractérisées en ce qu'elles contiennent des microcapsules selon l'une quelconque des revendications 1 à 7.
